# EUROPÄISCHE PATENTANMELDUNG

(11) **EP 2 574 339 A1**
(43) Veröffentlichungstag der Anmeldung: **03.04.2013**
(21) Anmeldenummer: 11182906.5
(22) Anmeldetag: 27.09.2011
(51) Int. Cl.: A61K 31/353, A61K 31/405, A61K 31/455, A61K 45/06

(54) **Pharmazeutisches Präparat zur Behandlung von NADH-bedingten Erkrankungen**

(71) Anmelder: Huber, Johannes, 1130 Wien (AT); Hochegger, Paul, 1040 Wien (AT)
(72) Erfinder: Huber, Johannes, 1130 Wien (AT)
(74) Vertreter: Sonn & Partner Patentanwälte

(57) **Zusammenfassung**

Beschrieben wird ein pharmazeutisches Präparat, enthaltend jeweils unabhängig voneinander
- 1 mg bis 10 g, vorzugsweise 1 bis 1000 mg, noch bevorzugter 10 bis 500 mg, insbesondere 50 bis 300 mg, Epigallokatechingallat (EGCG),
- 1 bis 500 mg, vorzugsweise 10 bis 100 mg, insbesondere 20 bis 80 mg, Nikotinamid-Ribose (NR) und/oder 0,05 bis 10 g, vorzugsweise 0,1 bis 5 g, insbesondere 0,5 bis 3 g, Tryptophan.

## Beschreibung

Die vorliegende Erfindung betrifft ein pharmazeutisches Präparat zur Behandlung von NADH-bedingten Erkrankungen.

NADH-bedingte Erkrankungen sind Erkrankungen, die entweder durch einen NADH-Mangel verursacht werden oder aber durch NADH-Gabe behandelbar sind oder verhindert werden können. Als besondere NADH-bedingte Erkrankungen sind hierfür Nervenerkrankungen oder Erkrankungen des Herz/Kreislaufsytems, insbesondere Bluthochdruck, zu nennen. Oft sind diese NADH-bedingte Erkrankungen durch einen Mangel an intrazellulären NADH bedingt oder können durch Erhöhung von intrazellulären NADH behandelt, verbessert oder verhindert werden. Beispiele hierfür sind in den US-Patenten US 4,970,200 A, US 5,444,053 A, US 5,668,114 A, US 5,712,259 A und US 5,736,529 A beschrieben, worin NADH-Gabe zur Behandlung/Verhinderung eine Reihe derartiger Erkrankungen herangezogen wurde. NADH ist jedoch sehr labil und nur schwer in die Zellen zu bringen, in denen NADH seine Wirkung entfalten soll.

Aufgabe der vorliegenden Erfindung ist, pharmazeutische Zusammensetzungen zur Verfügung zu stellen, die zur Behandlung von NADH-bedingten Erkrankungen, insbesondere von Nervenerkrankungen (z.B. Alzheimer'sche Erkrankung oder Parkinson'sche Erkrankung) und Herz-Kreislauferkrankungen (z.B. Bluthochdruck), geeignet sind.

Demgemäß betrifft die vorliegende Erfindung ein pharmazeutisches Präparat, enthaltend jeweils unabhängig voneinander
- 1 mg bis 10 g, vorzugsweise 1 bis 1000 mg, noch bevorzugter 10 bis 500 mg, insbesondere 50 bis 300 mg, Epigallokatechingallat (EGCG),
- 1 bis 500 mg, vorzugsweise 10 bis 100 mg, insbesondere 20 bis 80 mg, Nikotinamid-Ribose (NR) und/oder 0,05 bis 10 g, vorzugsweise 0,1 bis 5 g, insbesondere 0,5 bis 3 g, Tryptophan.

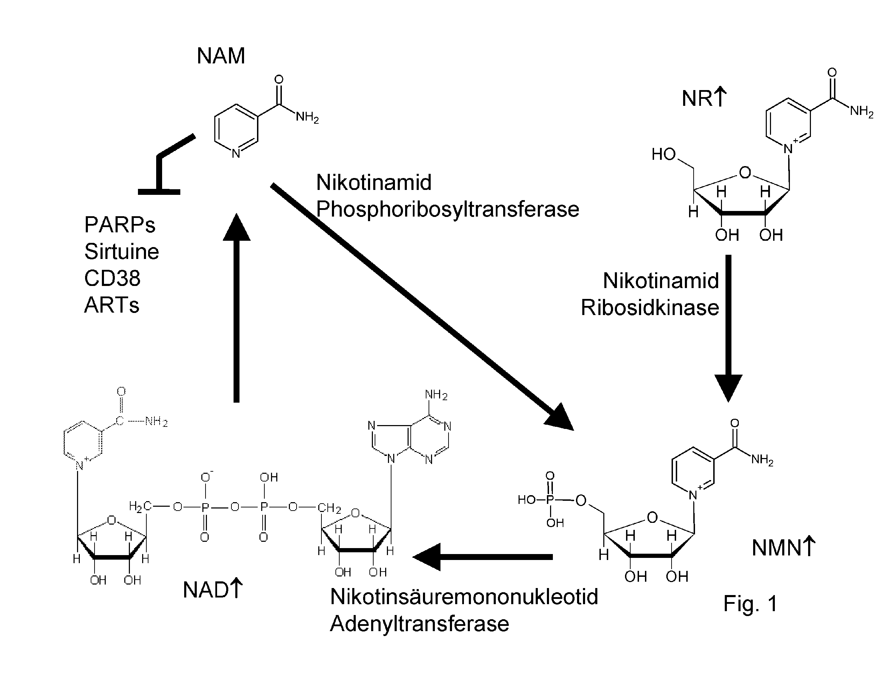

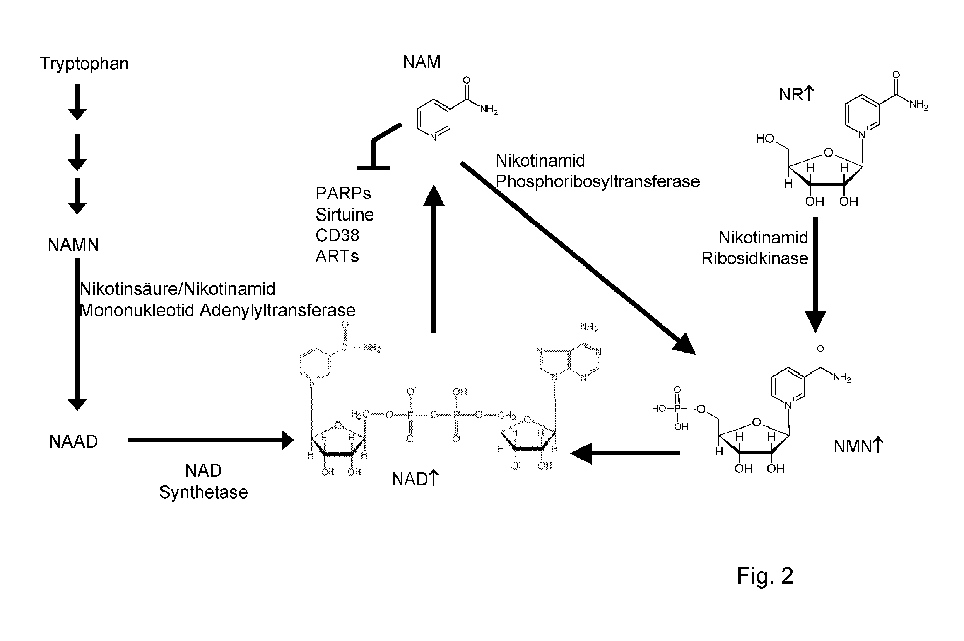

Epigallokatechingallat (EGCG; (2R,3R)-2-(3,4,5-Trihydroxyphenyl)-3,4-dihydro-1(2H)-benzopyran-3,5,7-triol-3-(3,4,5-trihydroxybenzoat)) ist ein natürlich vorkommendes Antioxidans. EGCG ist ein Katechin, das zur Untergruppe der Polyphenole zählt und in grünem Tee vorkommt. Neben vielen anderen Wirkungen weist EGCG einen Insulin-sedierenden Effekt sowie eine konzentrationssteigernde Wirkung auf. EGCG vermehrt die endogene Bildung des Nikotinsäureamid-Adenin-Dinukleotids (NAD (= NAD⁺)). Durch Gabe von EGCG wird die Nikotinamid-Ribosid-Kinase angeregt und vermehrt Beta-Nikotinamid-Mononukleotid gebildet. Das mit der Diät aufgenommene Tryptophan wird in der Zelle in Nikotinsäure-Mononukleotid (NAMN) umgewandelt, welches wieder durch Nikotinsäure/Nikotinamid-Mononukleotid Adenyltransferase in Nikotinsäure-Adenin-Dinukleotid (NAAD) umgewandelt wird, aus dem über die NAD Synthase das NAD entsteht. EGCG regt die NAD-Synthase sowie die Nikotinsäure/Nikotinamid Mononukleotid Adenyltransferase an, wodurch bei gleichzeitigem Vorhandensein des Tryptophans die endogene Bildung von NAD erhöht wird (siehe Fig. 1 und 2). EGCG wird vorzugsweise als Extrakt aus biologischen Quellen im erfindungsgemäßen Präparat vorgesehen, besonders bevorzugt als Extrakt aus Teepflanzen, insbesondere direkt aus dem grünen Tee oder nach dem Matcha-Verfahren gewonnen.

Mittel, welche EGCG entweder als Reinsubstanz oder als Bestandteil von grünem Tee enthalten und welche u.a. zur Behandlung von mentalen Krankheiten oder Befindlichkeitsstörungen eingesetzt werden, sind u.a. in JP 2003/286167 A, JP 11/018722 A, US 2002/086067 A1, US 2008/213401 A1 und WO 2008/006082 A2 beschrieben.

Auch die Nikotinamid-Ribose (NR; Nikotinamid-Ribosid) (1-[3,4-Dihydroxy-5-(hydroxymethyl)oxolan-2-yl]pyridin-5-carboxamid; auch als "Nikotinamid-Ribosid", "Nikotinamid-beta-Ribosid", "Nikotinamid-Ribonukleosid", "1-[(2R,3R,4S,5R)-3,4-Dihydroxy-5-(hydroxymethyl)oxolan-2-yl]pyridin-5-carboxamid", "N-Ribosylnikotinamid" bezeichnet (CAS: 1341-23-7)) ist ein Präkursor für das NAD und wird durch die Nikotinamid-Ribose-Kinase (nrk) in Nikotinamid-Mononukleotid umgewandelt, welches seinerseits über Nikotinamid Phosphoribosyltransferase (NNAD) in NAD konvertiert wird. Sowohl die Nikotinamid-Ribose-Kinase wie auch die Nikotinsäure-Nikotinamid-Mononukleotid-Adenyltransferase werden durch EGCG stimuliert, sodass die gleichzeitige Verabreichung von entweder Tryptophan oder Nikotinamid-Ribose mit EGCG zu einer verstärkten endogenen NAD-Synthase führt. Gleichzeitig wird dadurch auch die Aktivität der Sirtuine, die NAD abhängig ist, angeregt. Damit ist die Aktivität der Nikotinamid-Ribosidkinase mit der Aktivität der Sirtuine (vor allem Sirt 2) assoziiert. Im Zuge der vorliegenden Erfindung konnten auch Erkenntnisse über NR bei der zellulären NAD-Herstellung gewonnen werden, die NR als eine der zentralen Verbindungen in dieser Synthese ausweisen.

Tryptophan ((S)-2-Amino-3-(1H-indol-3-yl)propansäure) ist eine für den Menschen essentielle Aminosäure.

Wie EGCG können auch NR und Tryptophan sowohl in Form natürlicher Extrakte als auch in synthetischer Form (vgl. z.B. Yang et al., J. Med. Chem. 50 (2007): 6458 - 6461) erfindungsgemäß verwendet werden; eine andere vorteilhafte Variante ist die enzymatische Herstellung der Komponenten, insbesondere von NR. Bevorzugt sind in allen Fällen (z.B. aus natürlichen Quellen, aus Präparationen chemischer Synthesen oder Produkten aus enzymatischen Synthesen (oder Kombinationen derselben)) aufgereinigte Präparationen als Ausgangsstoffe zur Herstellung des erfindungsgemäßen Präparats (z.B. solche Extrakte oder Präparate, die EGCG und NR und/oder Tryptophan zu zumindest 10 Gew.-% enthalten, vorzugsweise zu zumindest 30 Gew.-%, insbesondere zu zumindest 50 Gew.-%, jeweils bezogen auf Trockengewicht). Die vorliegende Erfindung basiert auf der synergistischen Wirkung einer Kombination aus einer effektiven Menge an EGCG auf der einen Seite und einer effektiven Menge an NR oder an Tryptophan oder einer Kombination aus NR und Tryptophan auf der anderen Seite zur Bereitstellung eines Mittels zur Behandlung oder Prävention von NADH-bedingten Erkrankungen beim Menschen, insbesondere von Nervenerkrankungen oder Bluthochdruck. Mit der Kombination von EGCG und NR und/oder Tryptophan wird erfindungsgemäß eine Zusammensetzung zur Verfügung gestellt, die synergistische pharmazeutische Wirkung aufweist.

Unter "NADH-bedingten Erkrankungen" sind, wie eingangs erwähnt, Erkrankungen zu verstehen, die entweder durch einen NADH-Mangel verursacht werden oder aber durch NADH-Gabe behandelbar sind oder verhindert werden können. Als besondere NADH-bedingte Erkrankungen sind hierfür Nervenerkrankungen oder Erkrankungen des Herz/Kreislaufsytems, insbesondere Bluthochdruck, zu nennen. Oft sind diese NADH-bedingte Erkrankungen durch einen Mangel an intrazellulären NADH bedingt oder können durch Erhöhung von intrazellulären NADH behandelt, verbessert oder verhindert werden. Beispiele hierfür sind in den US-Patenten US 4,970,200 A (Parkinson'sche Erkrankung), US 5,444,053 A (Alzheimer'sche Erkrankung), US 5,668,114 A (Bluthochdruck), US 5,712,259 A (Chronisches Erschöpfungssyndrom), US 5,736,529 A (Schlaganfall oder andere akute neurale Traumata) und WO 2006/001982 A2 (Neuropathien oder Axonopathien, insbesondere neurodegenerative Erkrankungen, der "Motor Neuron Disease", Neoplasie, Nervenquetschung, demyelierende Erkrankungen, retinale oder optische Nervenstörungen, etc.) beschrieben. Auch wurde NADH als vorteilhaft bei der Tumorbehandlung, speziell, wenn diese mit einer Gabe von zytotoxischen Antitumor-Mitteln (Chemotherapie) einhergeht (WO 2006/001982 A2, WO 2006/056889 A2).

Ein vorrangiges Einsatzgebiet der vorliegenden Erfindung ist daher die Beeinträchtigung von Nervenfunktionen in Folge chronischer oder akuter Nervenstörungen. Bevorzugte Indikationen dabei sind die Parkinson'sche Erkrankung, die Alzheimer'sche Erkrankung, das Chronische Erschöpfungssyndrom (insbesondere soweit dessen neurale Symptome bekämpft oder verhindert werden), Schlaganfall oder andere akute neurale Traumata und Neuropathien oder Axonopathien, insbesondere neurodegenerative Erkrankungen, die "Motor Neuron Disease", Neoplasie, Nervenquetschungen, demyelierende Erkrankungen, retinale oder optische Nervenstörungen.

Ein weiteres prominentes Einsatzgebiet der vorliegenden Erfindung sind die Herz-Kreislauferkrankungen. Neben der Behandlung von Bluthochdruck und dem bereits oben erwähnten Schlaganfall sind hier auch die Behandlung von Herzinfarkten und Herzinsuffizienz zu nennen, wobei hier der vorbeugende Charakter der erfindungsgemäßen Verabreichung gesundheitsökonomisch fast noch wichtiger ist, als der Aspekt der Behandlung dieser Erkrankungen (insbesondere in der Akut-Phase).

Weiters findet die vorliegende Erfindung auch Anwendung im Rahmen von onkologischen Behandlungen, insbesondere, wenn die Behandlung der Tumorerkrankungen (insbesondere die Behandlung solider Tumoren) mit einer Chemotherapie (also mit zytotoxischen Agentien) einhergeht. Im Stand der Technik wurde die Vorteilhaftigkeit der NADH-Gabe insbesondere für Lungenkrebs (z.B. Adenokarzinom und auch non-small cell Lungenkrebs), Bauchspeicheldrüsenkrebs (z.B. exokrines Pankreas-Karzinom), Dickdarmkrebs (z.B. Kolonadenokarzinom und Kolonadenom), Prostatakrebs (einschließlich der fortgeschrittenen Erkrankung), hämatopoetische Tumoren der lymphatischen Linie (z.B. akute lymphatische Leukämie, B-Zell-Lymphom, das Burkitt-Lymphom), myeloische Leukämien (z.B. akute myeloische Leukämie (AML)), Schilddrüsenfollikelzellenkrebs, myelodysplastisches Syndroms (MDS), Tumoren mesenchymalen Ursprungs (z.B. Fibrosarkome und Rhabdomyosarkome), Melanome, Teratokarzinome, Neuroblastome, Gliome, gutartige Tumoren der Haut (z.B. Keratoakanthome), Brustkrebs (z.B. Brustkrebs im fortgeschrittenen Stadium), Nieren-Karzinom, Eierstock-Karzinom, Blasenkarzinom und epidermales Karzinom (WO 2006/056889 A2).

Daneben wurden auch andere angiogene Indikationen beschrieben, wie z.B. Makuladegeneration, Fettleibigkeit, Retinopathie, diabetische Retinopathie, Arthritis, rheumatoide Arthritis, Psoriasis und Restenose (WO 2006/056889 A2).

Vorzugsweise wird dem erfindungsgemäßen Präparat auch ein Süßstoff zugesetzt. Eine bevorzugte Variante enthält dabei den Süßstoff Steviosid (19-0-β-D-Glucopyranosyl-13-O-[β-D-glucopyranosyl(1→2)-β-D-glucopyranosyl]-steviol). Steviosid ist ein Diterpenglykosid und dreihundertmal süßer als Zucker. Steviosid kann aus Steviablättern (Stevia rebaudiana) gewonnen werden und ist für Diabetiker geeignet. Beim erfindungsgemäßen Präparat wird als Süßstoff vorzugsweise Stevia (rebaudiana) oder ein Steviaextrakt zugesetzt, welcher enzymatisch (fermentativ) behandelt sein kann.

Im erfindungsgemäßen Präparat werden vorzugsweise 1 bis 300 mg/l Steviosid vorgesehen. Steviablätter enthalten acht HauptGlykoside (Steviosid, Steviolbiosid, Rebaudiosid A, C, D, E und F sowie Dulcosid A), wobei Steviosid mit sechs bis achtzehn Prozent den größten Anteil an den in Steviablättern gefundenen Wirkstoffen hat. Vier dieser Steviol-Glykoside (Steviosid, Rebaudiosid A, Rebaudiosid C und Dulcosid) sind im Wesentlichen für die Süßwirkung verantwortlich; wovon Rebaudiosid A die besten sensorischen Eigenschaften aller vier Hauptglykoside aufweist, da es am süßesten und wenig bitter ist. Werden enzymatisch gewonnene Steviaprodukte erfindungsgemäß als Süßstoffe vorgesehen, so enthalten sie nahezu 100 % Rebaudioside und nur in geringen Spuren Steviosid. Vorteil dabei ist, dass das Getränk dann keinen bitteren Bei- oder Nachgeschmack aufweist. Rebaudiosid A wird als Süßstoff im erfindungsgemäßen Getränk vorzugsweise von 0,5 mg bis 200 mg vorgesehen (z.B. wenn enzymatisch gewonnene Stevia-Produkte zur Süßung verwendet werden). Hingegen ist bevorzugt, dass die erfindungsgemäße Zusammensetzung zuckerfrei verabreicht wird, da damit negative Wechselwirkungen mit Zucker (Glukose, Saccharose) verhindert werden.

Vorzugsweise enthält das erfindungsgemäße Präparat weitere Hilfs- und Geschmacksstoffe. Im Zusammenwirken mit EGCG und NR und/oder Tryptophan verhindert Steviosid die hyperinsulinämische Wirkung und unterstützt damit von einer anderen Seite den Effekt von EGCG.

Gemäß einer Variante der vorliegenden Erfindung kann das erfindungsgemäße Präparat in Trockenform vorgesehen werden, so dass es zur gebrauchsfertigen Form mittels Flüssigkeiten rekonstituiert werden kann (in pharmazeutischen Präparaten in der Regel eine pharmazutisch akzeptable Pufferlösung oder destilliertes (entionisiertes) Wasser (pharm. Grade); bei Präparaten, die vom Patienten selbst zubereitet werden in der Regel Wasser (insbesondere Mineralwasser), jedoch sind auch Fruchtsäfte, Limonaden und Ähnliches geeignet). Vorzugsweise ist dann die Trockenform in Einzeldosen, beispielsweise als Tagesdosen, abgepackt. Demgemäß beinhalten erfindungsgemäße Tagesdosen (z.B. in Sachets, Brausetabletten, etc.) beispielsweise (auch jeweils unabhängig voneinander) 1 mg bis 10 g, vorzugsweise 1 bis 1000 mg, noch bevorzugter 10 bis 500 mg, insbesondere 50 bis 300 mg, EGCG, 1 bis 500 mg, vorzugsweise 10 bis 100 mg, insbesondere 20 bis 80 mg, NR und/oder 0,05 bis 10 g, vorzugsweise 0,1 bis 5 g, insbesondere 0,5 bis 3 g, Tryptophan sowie gegebenenfalls 0,1 mg bis 500 mg, vorzugsweise 0,5 mg bis 200 mg, insbesondere 1 bis 100 mg, Steviosid. Eine derartige Trockenform (also ein Präparat in fester Form, insbesondere in Pulverform), kann auch z.B. in Form von Brausetabletten zur Verfügung gestellt werden. Entsprechende typisch verwendbare Hilfs- und Formulierungsstoffe oder Trägersubstanzen sind dem Fachmann auf diesem Gebiet bekannt und können bei Bedarf in den erfindungsgemäßen Mitteln entsprechend eingesetzt werden. Man kann die erfindungsgemäßen Präparate auch auf Basis höher konzentrierter Konzentrate (z.B. Sirups) zur Verfügung stellen, die dann entsprechend Vorgaben, z.B. 1:2 bis 1:20, vorzugsweise 1:5 bis 1:10, verdünnt werden können, um zu den erfindungsgemäßen Werten der Konzentrationen im Präparat zu gelangen.

Gemäß einer weiteren Ausführungsform enthält das erfindungsgemäße Präparat 200 bis 1000 mg/l EGCG und 80 bis 350 mg/l NR oder 0,5 bis 5 g/l Tryptophan.

Vorzugsweise wird das erfindungsgemäße Mittel auch als pharmazeutisches Präparat in Form von Arzneimitteldosierungen zur Verfügung gestellt. Die Herstellung dieser erfindungsgemäßen Präparate kann mit den üblichen Techniken/Zusatzstoffen vorgenommen werden, die dem Fachmann auf diesem Gebiet geläufig sind; insbesondere können natürlich die bereits für NADH bekannten Formulierungen ebenfalls herangezogen werden. Die erfindngsgemäßen Präparate können daher z.B. in Form von Tabletten, Kapseln, Dragees, etc. zur Verfügung gestellt werden. Dabei können diese mit magensaftresistenten Umhüllungen bereitgestellt werden. Vorzugsweise werden diese Arzneimitteldosierungen in Dosiseinheitsform, insbesondere als Tagesdosis (beispielsweise (auch jeweils unabhängig voneinander) 1 mg bis 10 g, vorzugsweise 1 bis 1000 mg, noch bevorzugter 10 bis 500 mg, insbesondere 50 bis 300 mg, EGCG, 1 bis 500 mg, vorzugsweise 10 bis 100 mg, insbesondere 20 bis 80 mg, NR und/oder 0,05 bis 10 g, vorzugsweise 0,1 bis 5 g, insbesondere 0,5 bis 3 g, Tryptophan sowie gegebenenfalls 0,1 mg bis 500 mg, vorzugsweise 0,5 mg bis 200 mg, insbesondere 1 bis 100 mg, Steviosid; nebst weiteren Inhaltsstoffen, wie pharmazeutischen Trägern oder Geschmacksstoffen), vorgesehen. Vorzugsweise enthalten die Tagesdosen 50 bis 200 mg EGCG und 20 bis 80 mg NR und/oder 0,5 bis 3 g Tryptophan sowie gegebenenfalls einen Süßstoff, insbesondere einen Süßstoff, umfassend Steviosid und/oder Rebaudiosid A (vorzugsweise in einer Menge von 1 bis 80 mg). Das erfindungsgemäße pharmazeutische Präparat kann insbesondere auch in Form eines Nahrungsergänzungsmittels zur Verfügung gestellt werden. Besonders bevorzugte Tagesdosierungen enthalten, unabhängig voneinander, 80 bis 150 mg, insbesondere 100 bis 130 mg, EGCG, 30 bis 70 mg, insbesondere 40 bis 60 mg, NR und/oder 1 bis 3 g Tryptophan. Diese Tagesdosen können natürlich, wie oben erwähnt, auch als Getränk zur Verfügung gestellt werden, vorzugsweise in einem Volumen von 100 bis 1000 ml, noch bevorzugter von 200 bis 750 ml, insbesondere 250 bis 500 ml.

Gemäß einem weiteren Aspekt betrifft die vorliegende Erfindung ein Kombinationspräparat, umfassend EGCG und NR (und/oder Tryptophan; wobei erfindungsgemäß die NR-Variante bevorzugt ist) als Wirksubstanzen, zur Verwendung bei der Behandlung von NADH-bedingten Erkrankungen, insbesondere Nervenerkrankungen und Bluthochdruck. Die Erfindung betrifft auch die Verwendung eines Kombinationspräparats, umfassend EGCG und NR als Wirksubstanzen, als Mittel zur Behandlung von NADH-bedingte Erkrankungen, insbesondere Nervenerkrankungen oder Bluthochdruck. EGCG kann prinzipiell zwar auch in einer Dosis von unter 1 mg eingenommen werden, jedoch muss dann die erfindungsgemäße Wirkung nicht unbedingt eintreten. Bei einer Dosis von über 10 g wird zwar die erforderliche Wirkung erzielt, es kann bei derart hohen Dosen aber zu Nebenwirkungen kommen, die unerwünscht sind, wie z.B. Einschlafproblemen oder anderen nervösen Begleiterscheinungen. Prinzipiell sind für die regelmäßige Einnahme eher geringere Dosierungen zu wählen; für die Einmaleinnahme zur Herbeiführung einer zeitnahen Verbesserung des Krankheitsbildes können eher höhere Dosen sowohl von EGCG als auch von NR (oder Tryptophan) genommen werden. Üblicherweise (auch dies jedoch oft abhängig von der Dosierung) tritt die therapeutische Wirkung etwa eine halbe Stunde bis eine Stunde nach der (oralen) Einnahme des erfindungsgemäßen Mittels ein und hält dann, je nach Dosis, über einen längeren Zeitraum, z.B. mehrere Stunden, an.

Demgemäß liegen bevorzugte Dosierungen für eine Einmaldosierung bei 10 mg bis 10 g EGCG und 1 bis 100 mg NR, vorzugsweise 100 mg bis 5 g EGCG und 2 bis 50 mg NR, insbesondere 500 mg bis 4 g EGCG und 5 bis 40 g NR (bzw. bei 10 mg bis 10 g EGCG und 0,1 bis 10 mg Tryptophan, vorzugsweise 100 mg bis 5 g EGCG und 0,5 bis 5 g Tryptophan, insbesondere 500 mg bis 4 g EGCG und 1 bis 4 g Tryptophan). Eine kontinuierliche Einnahme des erfindungsgemä-βen Mittels kann z.B. einmal täglich, einmal alle zwei Tage, einmal alle drei Tage erfolgen, jedoch können Einzeldosierungen auch mehrmals täglich (zweimal, dreimal, viermal, fünfmal, etc.) erfolgen. Dabei sollte darauf geachtet werden, dass einerseits die Dosis hoch genug ist, um einen erhöhten Level von EGCG und NR (bzw. Tryptophan) im Körper aufrechtzuerhalten; andererseits sollte man vor allem Überdosierungen von EGCG vermeiden (s. oben).

Die Einnahme des erfindungsgemäßen Präparates kann einerseits bereits in kombinierter Form erfolgen; andererseits kann das erfindungsgemäße Kombinationspräparat aber auch in getrennter Form eingenommen werden, z.B. kann die EGCG-Komponente in fester Form (z.B. als Pulver, Kapsel oder Tablette oral) und die NR-Komponente in Form einer wässrigen Lösung oder Suspension (z.B. ebenfalls oral) davor oder danach eingenommen werden. Demgemäß betrifft die vorliegende Erfindung auch ein Kombinationspräparat, umfassend EGCG und NR und/oder Tryptophan als Wirksubstanzen, insbesondere zur Verwendung bei der Behandlung von NADH-bedingten Erkrankungen, insbesondere Nervenerkrankungen und Bluthochdruck, wobei das Kombinationspräparat aus mindestens zwei getrennt voneinander einnehmbaren Komponenten besteht und in der einen Komponente EGCG und in der anderen Komponente NR und/oder Tryptophan vorgesehen wird.

Die Erfindung wird anhand der nachfolgenden Beispiele und der Zeichnungsfigur näher erläutert, ohne jedoch darauf eingeschränkt zu sein.

Es zeigen:
Fig. 1 und 2 den funktionellen Zusammenhang zwischen NR, NAD, Tryptophan und Sirtuinen;

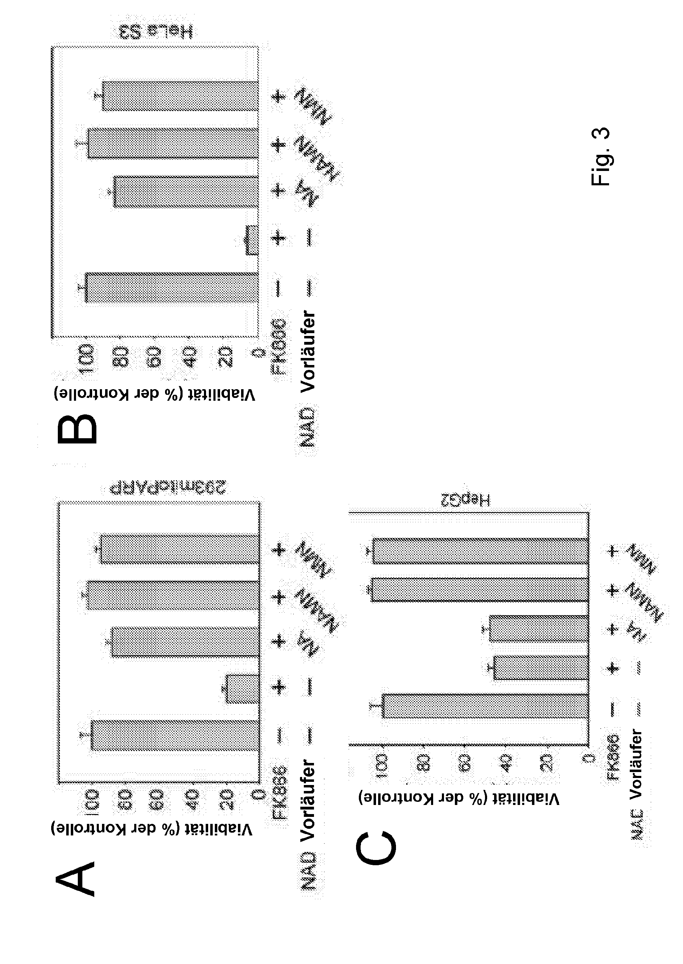

Fig. 3 die Unterstützung der mitochondrialen NAD⁺-Generierung und Überleben der Zellen durch extrazelluläre NAD⁺-Derivate; Nam Nutzung wurde von FK866 gehemmt; sowohl NAMN als auch NMN unterstützen mitochondriale NAD⁺-Bildung und schützen 293mitoPARP- (A) und HeLa S3-Zellen (B) vor FK866-induziertem Zelltod; Mononukleotid-Vorläufer, aber nicht NA, unterstützen die Generierung von mitochondrialem NAD⁺ und Lebensfähigkeit von HepG2-Zellen (C);

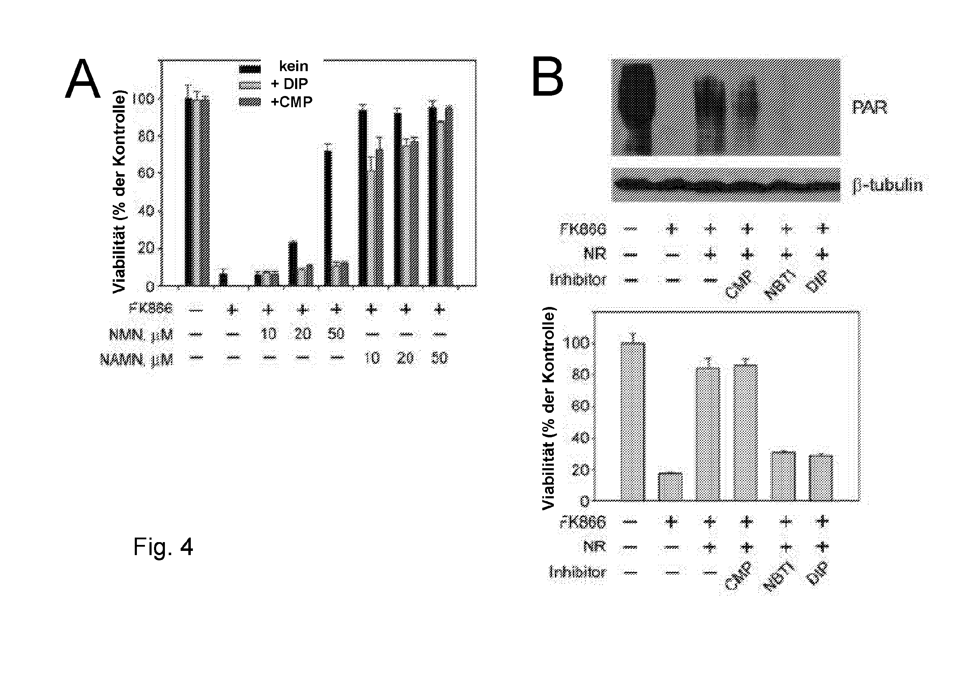

Fig. 4 den Abbau der extrazellulären Nukleotid-Vorstufen und die Aufnahme der resultierenden Riboside in die Zelle; wenn NMN oder NANM als NAD⁺-Vorstufen verwendet werden, hängt die Zellviabilität vom extrazellulären Abbau der Mononukleotide ab (A); extrazelluläres NR tritt in die Zelle über Nukleosid-Transporter ein und unterstützt die mitochondriale NAD⁺-Generierung und die Zellviabilität (B);

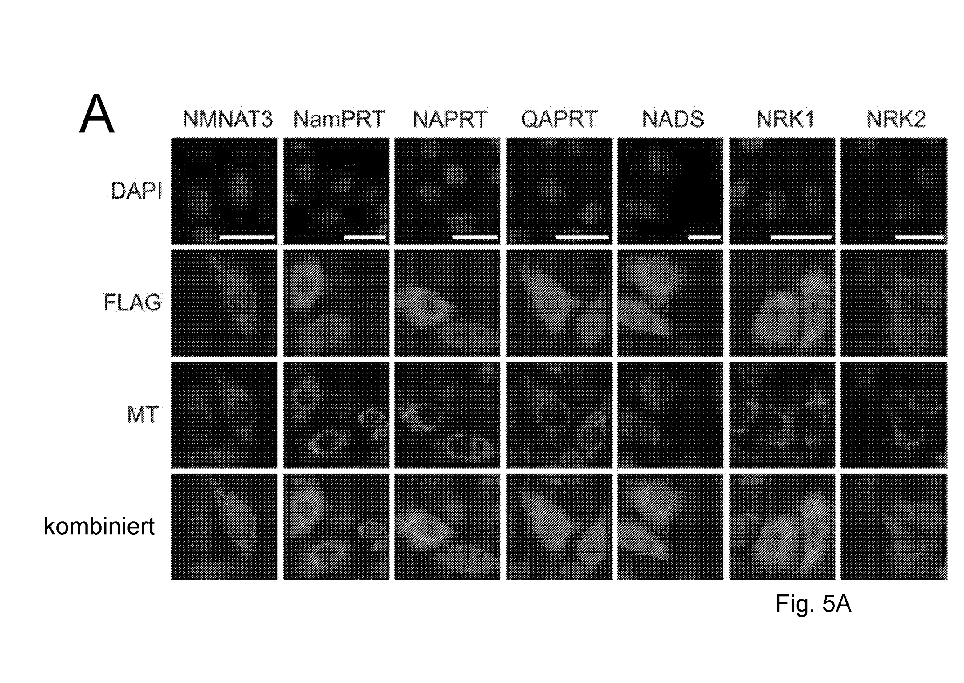

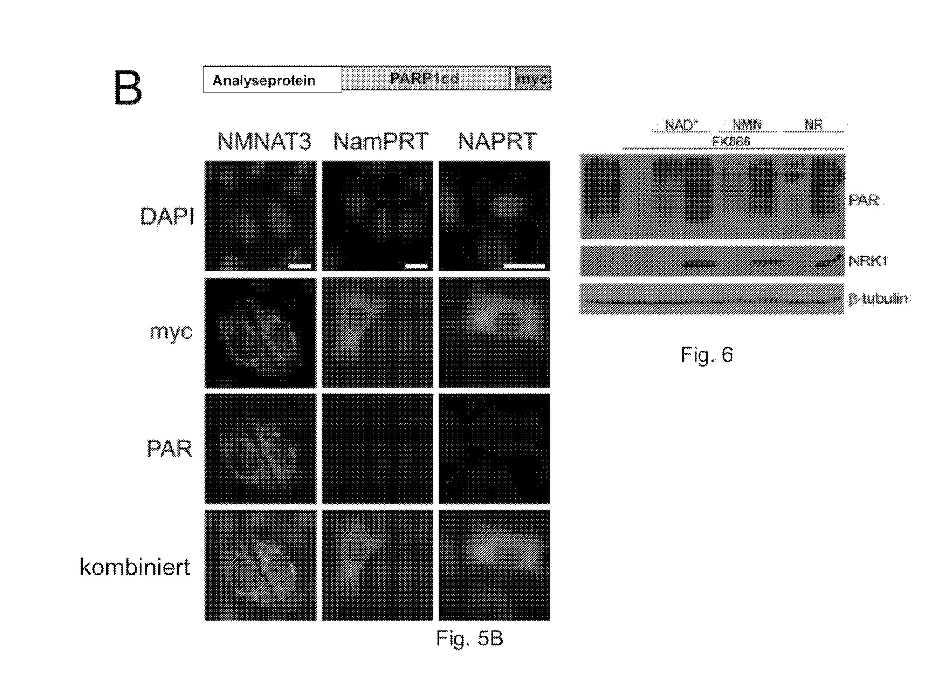

Fig. 5 die subzelluläre Lokalisation der NAD⁺-Biosyntheseenzyme; A: Alle bekannten NAD⁺-Biosyntheseenzyme lokalisieren im Zytoplasma oder Nukleus, mit Ausnahme der mitochondrialen NMNAT3; C-terminal FLAG-markierte Proteine wurden transient in HeLa S3 Zellen exprimiert; die Fluoreszenz-Aufnahmen zeigen Kerne (DAPI), exprimierte rekombinante Proteine (FLAG) und Mitochondrien (MT) (Balken: 20 µm) ; B: NMNAT3, aber nicht NamPRT oder NAPRT, ist ein mitochondriales Matrixprotein; submitochondriale Proteinlokalisation wurde nach Überexpression in HeLa S3 Zellen mit Hilfe von PARAPLAY durchgeführt; PAR Anhäufung zeigt Matrix-Lokalisation des Myc-Tag Analyt-PARP1cd Fusionsprotein (Balken: 20µm); und
Fig. 6 dass NMN die zytosolische Vorstufe mitochondrialen NAD⁺-Synthese ist; die Erhöhung des mitochondrialen NAD⁺-Gehalts durch Überexpression von NRK1 hängt von einer extrazellulären NR-Quelle ab (NMN, NAD⁺ oder NR, wie angegeben); zytosolisches NMN dient als Vorläufer; erhöhte NRK-Aktivität erhöht den mitochondrialen NAD⁺-Gehalt wenn NR, NMN oder NAD⁺ als extrazelluläre NAD⁺-Vorstufen vorgesehen werden.

### BEISPIELE:

### 1.: Herstellung

### 1.1.: Herstellung des erfindungsgemäßen pharmazeutischen Präparats (Tablette)

Eine direkte Kompression in 500mg Tabletten kann mit
- 100 mg EGCG,
- 10 mg NR,
- 50 mg Stevosid
- 10 mg SiO₂
- 20 mg Talkum
- Trägermittel (Mannitol) ad 500 mg
   vorgenommen werden.

### 1.2.: Herstellung des erfindungsgemäßen pharmazeutischen Präparats (Kapsel)

- 100 mg EGCG,
- 10 mg NR,
- 30 mg Na-Ascorbat
- 10 mg SiO₂
- 20 mg Talkum
- Trägermittel (mikrokristalline Zellulose oder Alginat) ad
   1g
   werden gemischt und in die vorgesehenen 1g Kapseln verfüllt.

### 1.3.: Herstellung des erfindungsgemäßen pharmazeutischen Präparats (Sachet)

- 100 mg EGCG,
- 10 mg NR,
- 30 mg Stevosid
- 10 mg SiO₂
- 20 mg Talkum
- Trägermittel (mikrokristalline Zellulose oder Alginat) ad 1g
- 3g Kalziumkarbonat
   werden gemischt und in 4g Sachets verpackt.

### 2.: Biochemische Charakterisierung

Die Wirksamkeit der erfindungsgemäßen Zusammensetzung kann auch biochemisch nachgewiesen werden, z.B. durch die in Dölle et al. (Anal. Biochem. (385) (2009): 377 - 379) und Berger et al. (J. Biol. Chem. 43 (280) (2005): 36334 - 36341) beschriebenen Methoden.

### 2.1.: Untersuchungen zur subzellulären Kompartimentierung der NAD-Biosynthese in menschlichen Zellen:

### 2.1.1.: Allgemeines:

Die Konzentration von NAD in Zellen und Geweben ist von entscheidender Bedeutung für die Aufrechterhaltung vitaler Funktionen. Zum einen ist NAD ein universeller Energieträger und damit essentiell für die Bereitstellung von ATP, dem Molekül, welches die Mehrzahl aller energieverbrauchenden Prozesse versorgt. Zum anderen ist NAD ein zelluläres Signalmolekül, welches durch spezifische Umwandlungen an der Regulation lebenswichtiger Ereignisse beteiligt ist. Dazu zählen u.a. die Regulation der Genexpression, der Zellteilung, der DNA-Reparatur sowie der Lebensspanne und des Langzeitgedächtnisses. Es ist bekannt, dass die Erhöhung der zellulären NAD Konzentration diese Prozesse positiv beeinflusst.

In menschlichen Zellen wird NAD aus Vitamin B3 synthetisiert. Vitamin B3 umfasst zwei sehr ähnliche Substanzen, Nikotinamid und Nikotinsäure (Nikotinat). Nikotinamid ist unter normalen Bedingungen ausreichend in der Nahrung vorhanden, so dass eine weitere Erhöhung (z.B. als Nahrungsergänzung) nahezu wirkungslos ist. Da Nikotinat über einen alternativen Syntheseweg zum NAD umgewandelt wird, würde eine erhöhte Zufuhr eine aussichtsreiche Strategie zur Erhöhung des NAD darstellen. In der Tat wird Nikotinat therapeutisch als lipid- (bzw. cholesterol-) senkendes Mittel angewendet, wobei diese Wirkungen durch rezeptorvermittelte Mechanismen erklärt werden. Diese Mechanismen scheinen auch die Ursachen für die erheblichen Nebenwirkungen zu sein, unter denen Hautreizungen, Ausschlag und Schwindelgefühl besonders häufig auftreten. Daher ist das Nikotinat ungeeignet, um als Nahrungsergänzung für die Erhöhung des NAD eingesetzt zu werden.

In der neueren Literatur wurde Nikotinamid-Ribosid (NR) als physiologisches Intermediat des NAD-Stoffwechsels beschrieben, welches auf einem alternativen Weg zum NAD umgesetzt wird.

Im Folgenden werden experimentelle Befunde vorgelegt, aus denen hervorgeht, dass NR tatsächlich von menschlichen Zellen aufgenommen und zu NAD umgesetzt werden kann. Obwohl zum Nahrungsgehalt des NR noch wenig bekannt ist, gibt es deutliche Hinweise, dass das NR dort nur in sehr geringen Mengen vorhanden ist. Mit den hierin dargestellten experimentellen Befunden wird aber nachgewiesen, dass mit NR die NAD Konzentration im Gewebe erhöht werden kann, ohne dabei die Nebenwirkungen des Nikotinats hervorzurufen. Weiters konnte beobachtet werden, dass die Zugabe von EGCG zu menschlichen Zellen eine Erhöhung der Expression der NMNAT1, einer Isoform des Enzyms NMNAT (Nikotinamid-Mononukleotid-Adenylyl-Transferase), die in allen Zellen gefunden wird, um 40% bewirken kann. Daraus ergibt sich auch eine physiologisch-wissenschaftliche Erklärung für die konzentrationssteigernde Wirkung des erfindungsgemäßen Getränkes, wobei offensichtlich die kombinierte Gabe von NR und EGCG die zelluläre NAD Synthese durch einen synergistischen Effekt positiv beeinflusst. Einerseits wird NR, zusätzlich zum Nikotinamid in der Nahrung, der NAD Synthese als alternative Vorläufersubstanz zugeführt. Andererseits wird durch EGCG eine notwendige Erhöhung der Kapazität des Enzyms herbeigeführt, welches an beiden Synthesewegen beteiligt ist.

### 2.1.2.: experimentelle Verfahren:

Klonieren und Erzeugung von eukaryontischen Expressionsvektoren
Für die Expression der getaggten Proteine, wurden die entsprechenden offenen Leserahmen in PFLAG-CMV-5a (Sigma), pcDNA3.1 (+)-PARP1cd (Dölle et al., Cell. Mol. Life Sci. 67 (2010), 433-443) oder pCMV/myc/mito (Invitrogen) eingefügt. Alle klonierten DNA-Sequenzen wurden durch DNA-Sequenzanalyse verifiziert.

### Pharmakologische Behandlungen

Inhibitoren (2 µM FK866, 2 mM 3-AB, 10 µM NBTI, 2 µM Dipyridamol, 2 mM CMP, 25 µM PPADS, 1 mM Ap4A) oder Metaboliten (100 µM NAD⁺, 100 µM NAAD, 100 µM NMN, 100 µM NAMN, 100 µM NA, 100 µM NR) wurden dem Zellkulturmedium zugesetzt, wie angegeben. NR wurde wie beschrieben bereitgestellt (Dölle et al., Anal. Biochem. 385 (2009), 377-379). Die Behandlung mit Inhibitoren und Metaboliten wurde für 24-48 h vor der Analyse von PAR Bildung und die Lebensfähigkeit der Zellen durchgeführt (72 h für 293mitoPARP Zellen, 96 h für HeLa S3-Zellen und 120 h für HepG2-Zellen). Die Lebensfähigkeit der Zellen wurde mittels MTT-Assay bestimmt.

### Immuncytochemie

Die Zellen wurden mit 4% (v/v) Formaldehyd in PBS fixiert und mit 0,5% (v/v) Triton X-100 in PBS permeabilisiert. Zellkerne wurden mit DAPI und Mitochondrien mit MitoTracker Red CMXRos (Invitrogen) gefärbt. Bilder wurden mit einem Leica DMI6000B Epifluoreszenz-Mikroskop (Leica Microsystems), ausgestattet mit x10, x40 und x100 Objektiven, aufgenommen.

Nachweis von Veränderungen im NAD⁺-Gehalt durch die PARP-Aktivität in Mitochondrien

Es wurden 293mitoPARP Zellen bereitgestellt, die ein Fusionsprotein exprimieren ("mitoPARP"), bestehend aus EGFP und PARP1cd (poly-ADP-Ribose-Polymerase-1), welches auf die mitochondriale Matrix abgezielt wurde. Mit mitochondrialem NAD⁺ als Substrat generieren diese Zellen konstitutiv Protein-gebundenes PAR (Protein-gebundene poly-ADP-Ribose), welches immunchemisch visualisiert wird. Variationen im Umfang der erfassten PAR spiegeln damit Veränderungen des mitochondrialen NAD⁺-Gehaltes wider. Ebenso wurde die transiente Expression von mitoPARP in HepG2 und HeLa S3 Zellen verwendet.

Identifizierung mitochondrialer Matrix-Proteine durch PARAPLAY

Der Poly-ADP-Ribose Assisted Protein Localisation Assay (PARAPLAY) liefert eine luminale Proteinlokalisation (Dölle et al., 2010). Hier wurde PARAPLAY zur Identifizierung mitochondrialer Matrixproteine verwendet. Die zu analysierenden Proteine (NMNAT3, NamPRT und NAPRT) wurden als N-terminale Fusionsproteine mit PARP1cd in HeLa S3 Zellen exprimiert. Aufgrund der niedrigen [NAD⁺] und/oder der hohen PAR-abbauenden Aktivität im Zytosol und im mitochondrialen Intermembranraum konnte kein PAR detektiert werden, wenn das Fusionsprotein in diesen Bereichen vorhanden ist. Im Gegensatz dazu ist Matrix-Lokalisierung leicht durch PAR-Akkumulation erkennbar. Auch wenn die Mehrheit des Fusionsproteins im Zytosol vorhanden ist, reicht ein luminaler Anteil des Proteins aus, um ausreichend immunologisch detektierbares PAR zu erzeugen. Wenn kein PAR detektiert werden kann, wird die Lokalisation des Proteins außerhalb der Matrix durch die Prüfung der Funktionalität des PARP1cd-Teils des Fusionsproteins verifiziert. Dies wird durch Zugabe einer N-terminalen mitochondrialen Targeting-Sequenz zum Fusionsprotein erreicht, womit es in die Matrix geleitet wird und zur PAR-Akkumulation führt.

### 2.1.3.: Resultate:

In situ Nachweis von relativen mitochondrialen NAD⁺-Spiegeln durch Poly-ADP-Ribose-Generierung in der Matrix

Eine gezielte Expression von PARP1cd in der mitochondrialen Matrix, hier an EGFP fusioniert und als "mitoPARP" bezeichnet, führt zu einer konstitutiven Anwesenheit von PAR innerhalb dieser Organellen. Ein PAR-Signal hängt sowohl von der katalytischen Aktivität von mitoPARP als auch von der Anwesenheit von NAD⁺ ab. Folglich erlaubt die mitoPARP-Expression die Erkennung von Änderungen im mitochondrialen NAD⁺-Gehalt. PAR war in keinem der Experimente im Zellkern nachweisbar, der Ort, an dem endogenes PARP-1 lokalisiert ist.

Identifizierung von extrazellulären NAD⁺-Metaboliten, welche die mitochondriale NAD⁺-Generierung unterstützen

Es wurde dann untersucht, welche extrazellulären NAD⁺-Vorstufen die mitochondriale NAD⁺-Generierung unterstützen. Jedenfalls erfüllt Nam diese Funktion, weil es in der Regel die einzige NAD⁺-Vorstufe in Zellkulturmedien ist. Wenn daher NamPRT gehemmt wird, muss eine alternative Vorstufe für die NAD⁺-Generierung vorhanden sein. Zugabe von Na, NAMN oder NMN zum Medium konnte den mitochondrialen NAD⁺-Gehalt und das Zell-Überleben wiederherstellen (Fig. 3A). Die Lebensfähigkeit der Zellen korreliert mit dem mitochondrialen NAD⁺-Spiegel, gemäß PAR-Messung. Transfizierte HeLa S3 Zellen zeigten eine ähnliche Empfindlichkeit gegenüber FK866 und Restaurierung des Überlebens durch NA, NAMN und NMN (Fig. 3B). Im Gegensatz dazu konnte NA in HepG2-Zellen keine Unterstützung für die Erzeugung von NAD⁺ und das Überleben der Zelle liefern (Fig. 3C) womit der Mangel an NA-Phosphoribosyltransferase-(NAPRT)-Aktivität in dieser Zelllinie bestätigt wurde.

Extrazelluläre Nukleotide werden zu den entsprechenden Nukleosiden abgebaut, die dann in die Zellen als NAD⁺-Vorstufen eintreten

Der NPP-Inhibitor PPADS verhindert die Wiederherstellung des mitochondrialen NAD⁺-Pools und das Überleben der Zelle, wenn NAD⁺ als extrazellulären Vorstufe verwendet wurde. Ebenso führte die Zugabe von Diadenosintetraphosphat (Ap4A) als kompetitives NPP-Substrat zu einem ähnlichen Effekt auf die Lebensfähigkeit der Zellen sowohl in 293mitoPARP als auch in und HeLa S3-Zellen. Um daher als Vorstufe zu dienen, muss extrazelluläres NAD⁺ zu NMN abgebaut werden. Der Zusatz von CMP (kompetitiert mit NMN als Substrat für die Dephosphorylierung durch die externe 5'-Nukleotidase) verringert die Zellviabilität von 293mitoPARP- und HeLa S3-Zellen deutlich. Weiters verlor NMN seine Funktion als extrazellulärer NAD⁺- Vorläufer auch in Gegenwart von Dipyridamol und NBTI, beides Inhibitoren des Plasmamembran-Nukleosid-Transporters. Die mitochondriale NAD⁺-Generierung von NAMN ist auch sensitiv gegenüber diesen Inhibitoren, wenn auch weniger stark (Fig. 4A). Diese Hemmung könnte durch eine Erhöhung der Konzentration von NAMN überwunden werden.

NR unterstützt die mitochondriale PAR-Bildung und Zelllebensfähigkeit in Gegenwart von CMP, während Dipyridamol und NBTI die NR-Nutzung hemmen (Fig. 4B). Aus diesen Ergebnissen kann daher geschlossen werden, dass neben NA und Nam, nur die Ribosid-Vorläufer NR und NAR als extrazelluläre Vorläufer des intrazellulären NAD⁺ dienen, während Mono-(NMN und NAMN) und Dinukleotide (NAD⁺ und NAAD) zunächst in die entsprechenden Nukleoside verarbeitet werden müssen.

Alle NAD⁺-Biosyntheseenzyme lokalisieren im Zytoplasma oder Nukleus, mit Ausnahme einer mitochondrialen NMNAT Isoform

Zum Verständnis der intrazellulären Signalwege der NAD⁺-Biosynthese wurden umfassende Analysen der subzellulären Verteilung der menschlichen Enzyme durchgeführt. Wenn diese Enzyme mit einem C-terminalen FLAG-Epitop in HeLa S3-Zellen exprimiert werden, lokalisieren alle Enzyme, ausgenommen NMNAT3, im Zytoplasma oder im Zellkern (Fig. 5A). NMNAT3 kolokalisiert mit Mitotracker, für die beiden anderen NMNAT Isoformen ist bekannt, dass sie an der nuklearen bzw. an der zytosolischen Seite des Golgi-Apparates lokalisieren.

Sowohl NamPRT als auch NAPRT erzeugen Substrate für NMNATs. Wenn diese in der vorliegenden Matrix vorhanden wären, könnten sie zur mitochondrialen NAD⁺-Synthese beitragen, vorausgesetzt NMNAT3 ist ein Matrix-Protein. Um diese Frage zu klären wurde der PARAPLAY-Assay angewendet, welcher speziell geeignet ist, suborganellare Proteinlokalisation aufzulösen (Dölle et al., 2010). Dieses Verfahren beinhaltet die Überexpression eines Analyt-Proteins, welches mit PARP1cd fusioniert ist. Für mitochondriale Proteine wird PAR-Akkumulation nur beobachtet, wenn sich das Fusionsprotein innerhalb der Matrix befindet. Daher wurden NMNAT3, NamPRT und NAPRT als Fusionsproteine mit PARP1cd exprimiert. Die Lokalisation dieser Konstrukte (Fig. 5B) war analog zu den entsprechenden FLAG-Proteinen (Fig. 5A): Das NMNAT3-PARP1cd-Protein unterstützt PAR-Akkumulation in Mitochondrien, wodurch ebenfalls belegt wird, dass NMNAT3 in der Tat eine Matrix-Protein ist. Weder das NamPRT- noch das NAPRT-PARP1cd-Fusionsprotein ergab eine nachweisbare Polymerbildung, wodurch belegt wird, dass diese keine Proteine der mitochondrialen Matrix in menschlichen Zellen sind.

Daraus folgt, dass NMNAT3 das einzige Enzym der NAD⁺-Synthese ist, welches in Mitochondrien menschlicher Zellen vorhanden ist. Alle anderen enzymatischen Aktivitäten der menschlichen NAD⁺-Biosynthese liegen im Kern und/oder im Zytoplasma. Daher ist klar, dass nukleares/zytosolisches NAD⁺ aus NR als extrazellulärer Vorstufe synthetisiert werden kann.

NMN ist die zytosolische Vorstufe der mitochondrialen NAD⁺-Synthese

Die Anwesenheit von nur NMNAT3 in der Matrix weist stark auf NMN als zytosolischen Vorläufer des mitochondrialen NAD⁺ hin. Die Abwesenheit der NAD-Synthetase (NADS) in Mitochondrien schließt die Möglichkeit aus, NA-Vorstufen innerhalb der Organellen zu amidieren. Darüber hinaus deutet die Lokalisation beider NRK Isoformen außerhalb Mitochondrien darauf hin, dass die Phosphorylierung von NR nicht innerhalb der Organellen auftritt.

Nach dem Eintritt in die Zelle, muss NR im Zytosol in NMN werden. In der Tat führt Überexpression von NRK1 (welches zytoplasmatisch ist) zu einer dramatischen Erhöhung der Menge an mitochondrialem PAR, aber nur, wenn ein extrazellulärer Vorläufer (wie NMN) zur Verfügung stand. Somit ist NR ein potenter Vorläufer der mitochondrialen NAD⁺, wenn dies im Zytosol an NMN phosphoryliert wird. Demgemäß wurde hiermit NMN funktionell als der zytosolische Vorläufer von mitochondrialem NAD⁺ bestätigt. Die Überexpression von NRK1 verbesserte erheblich die Verwendung von extrazellulärem NAD⁺ oder NR selbst zur mitochondrialen PAR-Bildung (Fig. 6). Diese Beobachtungen belegen, dass extrazelluläres NMN und NAD⁺ nicht in die Zellen aufgenommen werden, sondern vielmehr zunächst zu NR abgebaut werden müssen.

Dies erklärt auch die besonders schnelle Wirkung von NR im Rahmen der vorliegenden Erfindung bei der synergistischen Wirkung mit EGCG bei der Aktivierung dieser zellulären Prozesse.

## Patentansprüche

1. Pharmazeutisches Präparat zur Verwendung bei der Vorbeugung und Behandlung von NADH-bedingten Erkrankungen, enthaltend jeweils unabhängig voneinander
- 1 mg bis 10 g, vorzugsweise 1 bis 1000 mg, noch bevorzugter 10 bis 500 mg, insbesondere 50 bis 300 mg, Epigallokatechingallat (EGCG),
- 1 bis 500 mg, vorzugsweise 10 bis 100 mg, insbesondere 20 bis 80 mg, Nikotinamid-Ribose (NR) und/oder 0,05 bis 10 g, vorzugsweise 0,1 bis 5 g, insbesondere 0,5 bis 3 g, Tryptophan.

2. Präparat nach Anspruch 1, **dadurch gekennzeichnet, dass** es zusätzlich Steviosid enthält, vorzugsweise 0,1 bis 500 mg, noch bevorzugter 0,5 bis 200 mg, insbesondere 1 bis 100 mg, Steviosid.

3. Präparat nach Anspruch 1 oder 2, **dadurch gekennzeichnet, dass** es in fester Form, insbesondere in Pulverform, vorliegt.

4. Pharmazeutisches Präparat zur Verwendung bei der Vorbeugung und Behandlung von NADH-bedingten Erkrankungen in Dosiseinheitsform, enthaltend
- 1 mg bis 10 g, vorzugsweise 1 bis 1000 mg, noch bevorzugter 10 bis 500 mg, insbesondere 50 bis 300 mg, Epigallokatechingallat (EGCG),
- 1 bis 500 mg, vorzugsweise 10 bis 100 mg, insbesondere 20 bis 80 mg, Nikotinamid-Ribose (NR) und/oder 0,05 bis 10 g, vorzugsweise 0,1 bis 5 g, insbesondere 0,5 bis 3 g, Tryptophan.

5. Kombinationspräparat, umfassend EGCG und NR als Wirksubstanzen, zur Verwendung bei der Vorbeugung und Behandlung von NADH-bedingten Erkrankungen.

6. Kombinationspräparat nach Anspruch 5, **dadurch gekennzeichnet, dass** es 10 mg bis 10 g EGCG und 1 bis 100 mg NR, vorzugsweise 100 mg bis 5 g EGCG und 2 bis 50 mg NR, insbesondere 500 mg bis 4 g EGCG und 5 bis 40 g NR, umfasst.

7. Präparat nach einem der Ansprüche 1 bis 6, **dadurch gekennzeichnet, dass** die NADH-bedingte Erkrankung eine Nervenerkrankung ist, vorzugsweise eine akute oder chronischer Nervenstörung, insbesondere die Parkinson'sche Erkrankung, die Alzheimer'sche Erkrankung, das Chronische Erschöpfungssyndrom, Schlaganfall oder andere akute neurale Traumata und Neuropathien oder Axonopathien, neurodegenerative Erkrankungen, die "Motor Neuron Disease", Neoplasie, Nervenquetschungen, demyelierende Erkrankungen, retinale oder optische Nervenstörungen.

8. Präparat nach einem der Ansprüche 1 bis 6, **dadurch gekennzeichnet, dass** die NADH-bedingte Erkrankung eine Herz-Kreislauferkrankung ist, insbesondere Bluthochdruck, Schlaganfall, Herzinfarkt und Herzinsuffizienz.

9. Präparat nach einem der Ansprüche 1 bis 6, **dadurch gekennzeichnet, dass** die NADH-bedingte Erkrankung eine Tumorerkrankung ist, vorzugsweise Lungenkrebs, insbesondere Adenokarzinom und non-small cell Lungenkrebs, Bauchspeicheldrüsenkrebs, insbesondere exokrines Pankreas-Karzinom, Dickdarmkrebs, insbesondere Kolonadenokarzinom und Kolonadenom, Prostatakrebs, hämatopoetische Tumoren der lymphatischen Linie, insbesondere akute lymphatische Leukämie, B-Zell-Lymphom und das Burkitt-Lymphom, myeloische Leukämien, insbesondere akute myeloische Leukämie (AML), Schilddrüsenfollikelzellenkrebs, myelodysplastisches Syndrom (MDS), Tumoren mesenchymalen Ursprungs, insbesondere Fibrosarkome und Rhabdomyosarkome, Melanome, Teratokarzinome, Neuroblastome, Gliome, gutartige Tumoren der Haut, insbesondere Keratoakanthome, Brustkrebs, insbesondere Brustkrebs im fortgeschrittenen Stadium, Nieren-Karzinom, Eierstock-Karzinom, Blasenkarzinom und epidermales Karzinom.

10. Präparat nach einem der Ansprüche 1 bis 6, **dadurch gekennzeichnet, dass** die NADH-bedingte Erkrankung eine Makuladegeneration, Fettleibigkeit, Retinopathie, diabetische Retinopathie, Arthritis, rheumatoide Arthritis, Psoriasis oder Restenose ist.

11. Verwendung eines Kombinationspräparats, umfassend EGCG und NR als Wirksubstanzen, zur Verwendung bei der Vorbeugung und Behandlung von NADH-bedingten Erkrankungen.

12. Verwendung nach Anspruch 11, **dadurch gekennzeichnet, dass** das Kombinationspräparat als Getränk oder Getränkebasis oder als pharmazeutisches Präparat vorliegt.

13. Kombinationspräparat, umfassend EGCG und NR und/oder Tryptophan als Wirksubstanzen, zur Verwendung bei der Vorbeugung und Behandlung von NADH-bedingten Erkrankungen, wobei das Kombinationspräparat aus mindestens zwei getrennt voneinander einnehmbaren Komponenten besteht und in der einen Komponente EGCG und in der anderen Komponente NR und/oder Tryptophan vorgesehen wird.
